(19) Europäisches Patentamt
European Patent Office
Office européen des brevets

(11) Veröffentlichungsnummer: **0 187 248**
**B1**

# (12) EUROPÄISCHE PATENTSCHRIFT

(45) Veröffentlichungstag der Patentschrift: **08.06.88**

(51) Int. Cl.[4]: **C 08 G 63/62,** C 08 J 7/18, C 07 C 49/825, C 07 C 37/20

(21) Anmeldenummer: **85115134.0**

(22) Anmeldetag: **29.11.85**

## (54) Polycarbonate mit konjugierte Doppelbindungen enthaltenden Endgruppen, gegebenenfalls im Gemisch mit bekannten aromatischen Polycarbonaten, ihre Herstellung und ihre Verwendung.

(30) Priorität: **11.12.84 DE 3445108**

(43) Veröffentlichungstag der Anmeldung: **16.07.86 Patentblatt 86/29**

(45) Bekanntmachung des Hinweises auf die Patenterteilung: **08.06.88 Patentblatt 88/23**

(84) Benannte Vertragsstaaten: **DE FR GB IT NL**

(56) Entgegenhaltungen:
**EP-A-0 001 579**
**EP-A-0 102 573**

(73) Patentinhaber: **BAYER AG, Konzernverwaltung RP Patentabteilung, D-5090 Leverkusen 1 Bayerwerk (DE)**

(72) Erfinder: **Wehnert, Wolfgang, Dr., Bodelschwinghstrasse 14, D-4150 Krefeld (DE)**
Erfinder: **Bottenbruch, Ludwig, Dr., Wöhlerstrasse 5, D-4150 Krefeld (DE)**
Erfinder: **Löwer, Hartmut Mobay Chemical Corporation, Plastic Division Mobay Road, Pittsburgh, PA 15205 (US)**

**EP 0 187 248 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Gegenstand der vorliegenden Erfindung ist ein Verfahren zur Herstellung von aromatischen Polycarbonaten mit konjugierte Doppelbindungen enthaltenden Endgruppen und mit mittleren Molekulargewichten $\bar{M}_w$ (Gewichtsmittel, ermittelt durch Lichtstreuung) zwischen 2000 und 100 000, vorzugsweise zwischen 5000 und 80 000, besonders bevorzugt zwischen 10 000 und 50 000, gegebenenfalls im Gemisch mit bekannten aromatischen Polycarbonaten mit $\bar{M}_w$ (Gewichtsmittel, ermittelt durch Lichtstreuung) zwischen 2000 und 100 000, vorzugsweise zwischen 5000 und 80 000, besonders bevorzugt zwischen 10 000 und 50 000, aus Diphenolen, 0,5 bis 40 Mol-%, vorzugsweise 1 bis 20 Mol-%, besonders bevorzugt 2 bis 10 Mol-%, bezogen auf Mole Diphenole, Kettenabbrechern und Phosgen nach den bekannten Polycarbonatherstellungsmethoden des Phasengrenzflächenverfahrens oder des Verfahrens in homogener Lösung, das dadurch gekennzeichnet ist, daß man Kettenabbrecher der Formel (Ia) oder (Ib)

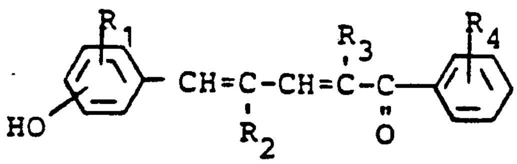

(Ia)

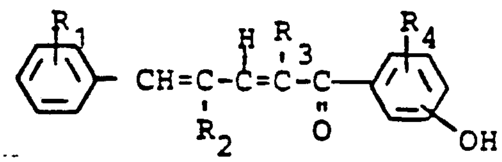

(Ib),

gegebenenfalls in Kombination mit maximal gleichen Molmengen an anderen, bekannten Kettenabbrechern verwendet.

In den Kettenabbrechern der Formel (Ia) und (Ib) sind $R_1$ bis $R_4$ gleich oder verschieden und H, $CH_3$, $C_2H_5$, n-$C_3H_7$, iso-$C_3H_7$, n-$C_4H_9$, iso-$C_4H_9$, sec-$C_4H_9$, $C_6H_5$ oder Cl.

Bekannte, bei dem erfindungsgemäßen Verfahren mitzuverwendende andere Kettenabbrecher sind beispielsweise Phenole, Carbonsäurehalogenide, Sulfonsäurechloride oder Chlorkohlensäureester, vorzugsweise jedoch Phenole.

Beispiele für die bekannten, mitzuverwendenden Kettenabbrecher sind Phenol, p-tert.-Butylphenol, 2,6-Dimethylphenol oder p-Isooctylphenol.

Gegenstand der vorliegenden Erfindung sind außerdem die nach dem erfindungsgemäßen Verfahren erhältlichen aromatischen Polycarbonate mit $\bar{M}_w$ zwischen 2000 und 100 000 mit konjugierte Doppelbindungen enthaltenden Endgruppen der Formel (Ic) oder (Id)

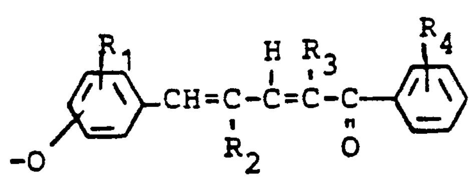

(Ic)

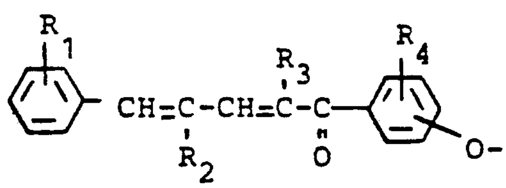

(Id)

worin $R_1$ bis $R_4$ die für die Formel (Ia/Ib) genannte Bedeutung haben, gegebenenfalls im Gemisch mit bekannten aromatischen Polycarbonaten, mit $\bar{M}_w$ zwischen 2000 und 100 000, die aus dem Kettenabbruch mit den anderen, bekannten Kettenabbrechern resultieren.

Erfindungsgemäß erhältliche, aromatische Polycarbonate mit mittleren Molekulargewichten $\bar{M}_w$ (Gewichtsmittel ermittelt durch Lichtstreuung) zwischen 2000 und 100 000 sind vorzugsweise solche der Formel (II)

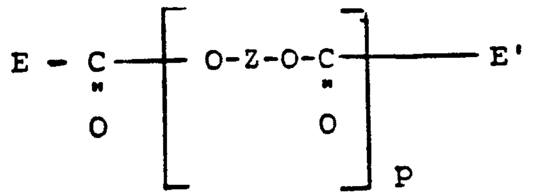 (II)

worin -O-Z-O- ein Diphenolatrest mit vorzugsweise 6 bis 30 C-Atomen ist, E und E' gleich oder verschieden sind und wobei mindestens einer der Reste E oder E' einem Rest der Formel (Ic) oder (Id) entspricht, worin $R_1$ bis $R_4$ die für Formel (Ia/Ib) genannte Bedeutung haben, und wobei die übrigen Endgruppen E und E' aus der Reaktion mit den anderen, bekannten, phenolischen Kettenabbrechern resultieren und wobei "p" der Polymerisationsgrad ist, der aus den Molekulargewichten $\bar{M}w$ von 2000 bis 100 000 resultiert.

Die erfindungsgemäßen Polycarbonate besitzen aufgrund der Endgruppen die Fähigkeit, beim Erhitzen, also bei einer Wärmebehandlung, insbesondere aber beim Bestrahlen mit ultraviolettem und/oder sichtbarem Licht ihr Molekulargewicht zu erhörhen. Die Wärmebehandlung kann auch durch IR-Bestrahlung erfolgen, die Belichtung auch durch Sonnenlicht bei natürlicher Bewitterung.

Thermoplastische, aromatische Polycarbonate mit ungesättigten Endgruppen sind bekannt (siehe Deutsche Offenlegungsschriften Nr. 2 746 139 (Le A 18 392), 2 829 256 (Le A 18 847), 2 843 154 (Le A 19 147) und 2 824 004 (Le A 19 148). Die Doppelbindungen dieser Polycarbonate können für bestimmte Reaktionen (Pfropfung, UV-Lichtvernetzung) genutzt werden. Demgegenüber erfolgt bei Belichtung der erfindungsgemäß erhältlichen Polycarbonate ein Molekulargewichtsaufbau ohne Vernetzung. Die belichteten beziehungsweise thermisch behandelten Polycarbonate sind somit noch in Polycarbonatlösungsmitteln in den bekannten Konzentrationen, beispielsweise in 10 %-iger Lösung in $CH_2Cl_2$, löslich.

Aus der DE-OS 3 232 391 (Le A 21 741) sind derartige Polycarbonate im Prinzip bekannt, die mit Kettenabbrechern der allgemeinen Formel

$X\text{-}A\text{-}(CH_2)_n\text{-}CH{=}CH\text{-}CH{=}CH\text{-}R$

hergestellt werden, worin R ein aromatischer Rest oder ein Alkyl-Rest sein kann, n eine Zahl von 0 bis 7 und -A- eine Einfachbindung, oder X-A ein

Rest 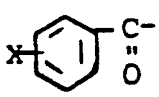 oder 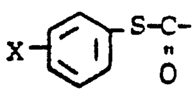 sein können und worin X

vorzugsweise auch OH sein kann.

Es war jedoch nicht vorhersehbar, daß im Falle von

R = Phenyl, X-A = HO 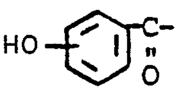 und "n" = O Polycarbonate

resultieren, die neben einem thermischen Molekulargewichtsaufbau überraschend auch einen unvernetzten photochemischen Aufbau ermöglichen.

Die Kettenabbrecher der Formel (Ia) und (Ib) sind unseres Erachtens in der Literatur noch nicht beschrieben. Sie lassen sich nach folgendem Verfahren synthetisieren:

In eine Lösung von überschüssigem NaOH in etwa 33 Vol-%-igem Ethanol werden bei Raumtemperatur Zimtaldehyd oder ein entsprechendes Derivat des Zimtaldehyds sowie Acetophenon oder ein entsprechendes Acetophenon-Derivat eingetragen, von denen eines die phenolische OH-Gruppe trägt.

Nach zweistündiger Umsetzung bei 50°C wird mit dem gleichen Volumen Wasser verdünnt und mit konzentrierter Salzsäure angesäuert.

Das Produkt wird mit Methylenchlorid extrahiert, die organische Phase mit Wasser neutral gewaschen, eingeengt und der anfallende Rückstand umkristallisiert.

Die Reaktion läßt sich durch folgende Gleichung veranschaulichen

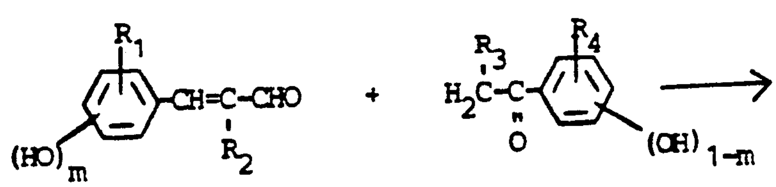 I (a/b)

(m = 1 oder 0) (NaOH/$C_2H_5OH$/$H_2O$)
50° C/2 Stunden
(Ie) (If)

Gegenstand der vorliegenden Erfindung sind somit auch Verbindungen der Formel I(a/b)

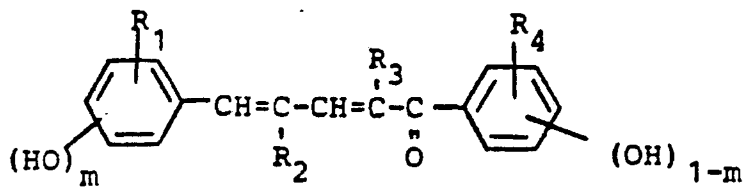

worin $R_1$ bis $R_4$ gleich oder verschieden sind und H, $CH_3$, $C_2H_5$, n-$C_3H_7$, iso-$C_3H_7$, n-$C_4H_9$, iso-$C_4H_9$ sec.-$C_4H_9$, $C_6H_5$ oder Cl sein können und "m" 0 oder 1 sein kann.

Gegenstand der vorliegenden Erfindung ist außerdem ein Verfahren zur Herstellung der Verbindungen der Formel I (a/b), das dadurch gekennzeichnet ist, daß man Zimtaldehyde der Formel (Ie) mit Acetophenonen der Formel (If) in Alkohol/Wasser-Gemischen, vorzugsweise in Gemischen von 1 Volumenteil Alkohol und 2 Volumenteilen Wasser, bei Temperaturen von 20°C bis 100°C, vorzugsweise von 30°C bis 80°C, und Reaktionszeiten von etwa 30 Minuten bis etwa 3 Stunden umsetzt, danach das erhaltene Reaktionsgemisch ansäuert, mit einem organischen, nicht mit Wasser mischbaren Lösungsmittel, vorzugsweise mit $CH_2Cl_2$, extrahiert und den Extrakt in bekannter Weise aufarbeitet.

Verbindungen der Formel (Ia) sind beispielsweise

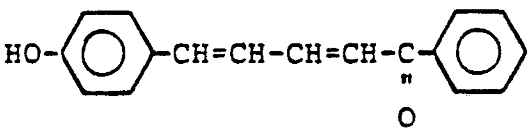

und

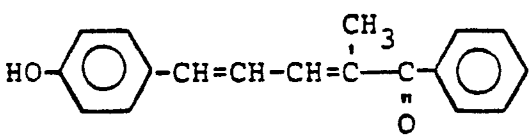

.

Sie lassen sich nach obigem Verfahren herstellen.

Verbindungen der Formel (Ib) sind beispielsweise

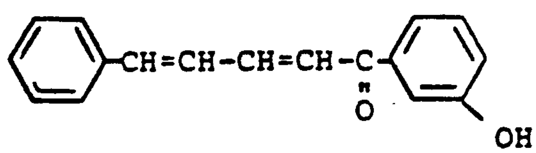

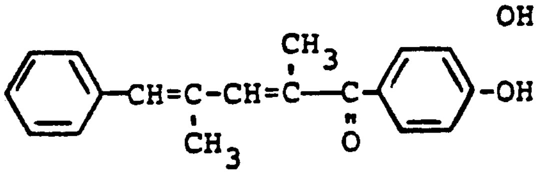

und

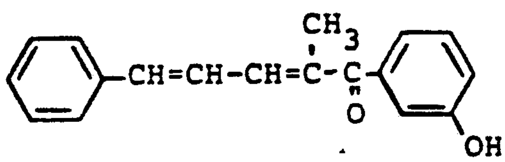

.

Sie lassen sich nach obigem Verfahren herstellen.

Für die Herstellung der erfindungsgemäßen Polycarbonate geeignete Diphenole der Formel (III)

HO-Z-OH (III)

mit vorzugsweise 6 bis 30 C-Atomen sind sowohl einkernige, als auch mehrkernige Diphenole, die Heteroatome enthalten können und unter den Bedingungen der Polycarbonatherstellung und deren thermischer oder photochemischer Behandlung inerte Substituenten haben können.

Beispielsweise seien Hydrochinon, Resorcin, Dihydroxydiphenyl, Bis-(hydroxyphenyl)-alkane, Bis-(hydroxyphenyl)-cycloalkane, Bis-(hydroxyphenyl)-sulfide, -ether, -ketone, -sulfoxide, -sulfone und α α'-Bis-(hydroxyphenyl)-diisopropylbenzole sowie deren kernalkylierte und kernhalogenierte Verbindungen genannt.

Geeignete Diphenole sind beispielsweise in den US-Patenten 3 028 365, 2 999 835, 3 062 781 und 3 148 172, in den Deutschen Offenlegungsschriften 1 570 703 und 2 063 050 sowie in der Monographie "H. Schnell, Chemistry and Physics of Polycarbonates, Interscience Publishers, New York, 1964" beschrieben.

Bevorzugte Diphenole sind

4,4'-Dihydroxydiphenyl, 2,2-Bis-(4-hydroxyphenyl)-propan, 2,4-Bis-(4-hydroxyphenyl)-2-methylbutan, 1,1-Bis-(4-hydroxyphenyl)-cyclohexan, α,α'-Bis- (4-hydroxyphenyl)-p-diisopropylbenzol, 2,2-Bis-(3-methyl-4-hydroxyphenyl)-propan, 2,2-Bis-(3-chlor-4-hydroxy-phenyl)-propan, Bis-(3,5-dimethyl-4-hydroxyphenyl)-methan, 2,2-Bis- (3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,1-Bis-(3,5-dimethyl-4-hydroxyphenyl)-cyclohexan, α,α'-Bis-(3,5-dimethyl-4-hydroxyphenyl)-p-diisopropylbenzol, 2,2-Bis-(3,5-dichlor-4-hydroxyphenyl)-propan und 2,2-Bis-(3, 5-dibrom-4-hydroxyphenyl)-propan.

Besonders bevorzugte Diphenole sind z.B.: 2,2-Bis-(4-hydroxyphenyl)-propan 2,2-Bis-(3,5-dimethyl-4-hydroxyphenyl)-propan 2,2-Bis- (3,5-dichlor-4-hydroxyphenyl)-propan 2,2-Bis- (3,5-dibrom-4-hydroxyphenyl)-propan 1,1-Bis-(4-hydroxyphenyl)-cyclohexan.

Es können auch beliebige Mischungen der vorgenannten Diphenole verwendet werden.

Neben den genannten monomeren Diphenolen können zur Darstellung von Blockcopolycarbonaten mit den erfindungsgemäßen Endgruppen auch Oligomere oder Polymere mitverwendet werden, die zwei unter den Bedingungen der Polycarbonatsynthese zur Aufbaureaktion befähigte Endgruppen tragen. Solche Endgruppen sind z. B. phenolische OH-Gruppen, Chlorkohlensäureestergruppen und Carbonsäurechloridgruppen. Vorgefertigte oligomere oder polymere Blöcke, die die genannten reaktiven Gruppen infolge der Art ihrer Herstellung bereits tragen oder bei denen man solche Gruppen durch eine geeignete Nachbehandlung erzeugen kann, sind z. B. Polysiloxane, Polykondensate auf der Basis von aliphatischen Diolen und gesättigten aliphatischen oder aromatischen Dicarbonsäuren, wie beispielsweise gesättigte, aliphatische Polyester auf der Basis von hydrierter Dimerfettsäure, aromatische Polyethersulfone und aliphatische Polyether.

Derartige cokondensierbare, bifunktionelle Oligomere oder Polymere können Molekulargewichte $\bar{M}n$ (Zahlenmittel bestimmt durch Dampfdruckosmose) zwischen 500 und 10 000 haben und werden in solchen Mengen eingesetzt, daß die resultierenden erfindungsgemäßen, ungesättigte Endgruppen enthaltenden Polycarbonate bis zu 80 Gew.-%, vorzugsweise zwischen 2 und 20 Gew.-%, davon enthalten.

Gegenstand der vorliegenden Erfindung ist somit auch die Modifizierung des erfindungsgemäßen Herstellungsverfahrens, das dadurch gekennzeichnet ist, daß man unter den Polycarbonatherstellungsbedingungen cokondensierbare bifunktionelle Oligomere oder Polymere mit Molekulargewichten $\bar{M}n$ (Zahlenmittel) zwischen 500 und 10 000 in Mengen bis zu 450 Gew.-%, vorzugsweise zwischen etwa 3 Gew.-% und 30 Gew.-%, bezogen auf Gewichtsmenge an eingesetzten monomeren Diphenolen, mitverwendet.

Gegenstand der vorliegenden Erfindung sind somit auch die nach diesem modifizierten, erfindungsgemäßen Verfahren erhältlichen aromatischen segmentierten Polycarbonate mit konjugierte Doppelbindungen enthaltenden Endgruppen der Formel (Ic) oder (Id), gegebenenfalls im Gemisch mit bekannten aromatischen segmentierten Polycarbonaten, die aus dem Kettenabbruch mit den anderen, bekannten Kettenabbrechern resultieren.

Die erfindungsgemäß erhältlichen Polycarbonate bzw. Polycarbonatgemische können in bekannter Weise durch den Einbau von geringen Mengen an Verzweigern verzweigt sein, um in bekannter Weise das Fließverhalten zu verbessern.

Die Herstellung der erfindungsgemäßen Polycarbonate bzw Polycarbonatgemische kann im wesentlichen nach den beiden bekannten Verfahren, dem Zweiphasengrenzflächenverfahren oder dem sogenannten Pyridinverfahren erfolgen (vgl. H. Schnell, "Chemistry and Physics of Polycarbonates", Polymer Reviews, Vol. IX, Seite 27 ff., Interscience Pub. 1964). Bevorzugt wird das Zweiphasengrenzflächen-Verfahren.

Hierbei werden die einzusetzenden Diphenole in wäßriger alkalischer Phase gelöst. Dazu werden die zur Herstellung der erfindungsgemäßen Polycarbonate erforderlichen Kettenabbrecher in Mengen von 0,5-40,0 Mol-%, bezogen auf Mole Diphenol, in einem organischen Lösungsmittel gelöst oder in Substanz, zugegeben. Dann wird in Gegenwart einer inerten, vorzugsweise Polycarbonat-lösenden, organischen Phase mit Phosgen umgesetzt. Die Reaktionstemperatur liegt zwischen 0° C und 40° C.

Als organische Phase kommen in bekannter Weise $CH_2Cl_2$, Chlorbenzol oder Gemische dieser Lösungsmittel in Betracht.

Um den Umsatz der Polykondensation und damit auch den Polymerisationsgrad des resultierenden Produkts zu erhöhen, kann ein im Verlauf der Umsetzung abgesunkener pH-Wert durch Zugabe von weiterem Alkali wieder angehoben werden.

Die Zugabe der erforderlichen Kettenabbrecher, in Art und Menge wie oben angegeben, kann auch während der Phosgenierung erfolgen.

Geeignete organische Lösungsmittel für die Kettenabbrecher sind beispielsweise Methylenchlorid, Chlorbenzol, Mischungen aus Methylenchlorid und Chlorbenzol, Aceton, Acetonitril, Toluol.

Die Reaktion kann durch Katalysatoren wie Tributylamin oder Triethylamin begünstigt werden. Um den Einbau des Kettenabbrechers zu begünstigen, können auch Oniumsalze, wie etwa Tetraalkylammoniumhalogenide als Phasentransferkatalysatoren mitverwendet werden.

Werden Verzweiger bzw. cokondensierbare bifunktionelle Oligomere oder Polymere mitverwendet, so kann deren Zugabe vor der Umsetzung mit Phosgen oder während der Phosgenierung erfolgen.

Neben oder anstelle der Diphenole können auch deren Chlorkohlensäureester eingesetzt werden.

Die Isolierung de erfindungsgemäßen Polycarbonate erfolgt in bekannter Weise. Um bei der Aufarbeitung nicht schon einen teilweisen Aufbau des Molekulargewichtes herbeizuführen, sollten Temperaturen größer 100°C nach Möglichkeit nicht angewendet werden. Geeignete Aufarbeitungsverfahren sind insbesondere das Ausfällen, die Sprühtrocknung und das Verdampfen des Lösungsmittels im Vakuum.

Die Formgebung der erfindungsgemäß erhältlichen Polycarbonate bzw. Polycarbonatgemische kann durch thermoplastische Verarbeitungsverfahren, z. B. durch Extrusion oder Spritzgießen, sowie durch das Eindampfen von Polymerlösungen, z. B. beim Folien-Gießverfahren, erfolgen. Bei thermoplastischer Verarbeitung muß jedoch beachtet werden, daß man einerseits von genügend niedrigviskosen Produkten ausgeht und andererseits den Durchsatz, beispielsweise im Extruder, so einstellt, daß der Molekulargewichtsanstieg kontrollierbar bleibt.

Die erfindungsgemäßen Polycarbonate können durch Erwärmen auf Temperaturen zwischen 150°C bis 400°C, vorzugsweise auf Temperaturen zwischen 200°C bis 350°C, in unvernetzte Polycarbonate mit höherem Molekulargewicht überführt werden. Dies gilt auch für verzweigte Produkte, sofern sie höchstens zwei erfindungsgemäße Endgruppen pro Polymermolekül enthalten. Die Dauer der Wärmebehandlung richtet sich nach der eingestellten Maximaltemperatur und dem gewünschten Molekulargewichtsaufbau. Die Wärmebehandlung wird mit Vorteil bei der Verarbeitung zu Formkörpern in Extrudern, Pressen oder Spritzgußmaschinen durchgeführt. Sie kann auch durch Aufschmelzen in Knetern oder Rührkesseln erfolgen. Das Ausmaß der Molekulargewichtserhöhung ist z. B. durch einen Vergleich der Lösungsviskosität der Probe vor und nach der Wärmebehandlung feststellbar

Das Bestrahlen der erfindungsgemäß erhältlichen Polycarbonate bzw Polycarbonatgemische erfolgt entweder nach der Formgebung an Formkörpern oder in Lösungen, welche anschließend zu Folien vergossen werden können. Die Bestrahlung wird durchgeführt, indem man entweder eine solche Lösung mit einer Tauchlampe bestrahlt, z. B. eine 10 %-ige Polycarbonat-Lösung in Methylenchlorid mit einer Quecksilbertauchlampe, oder aber Fasern, Folien oder Formteile mit einer UV-Lampe bestrahlt.

Die resultierenden Polycarbonate sind, ausgehend von linearen Produkten, jeweils noch in Polycarbonatlösungsmitteln lösliche Thermoplaste; ausgehend von verzweigten Verbindungen sind die entstehenden Polymeren ebenfalls löslich, wenn man verzweigte Produkte mit einem mittleren Gehalt von höchstens 2,0 erfingsgemäßen Endgruppen pro Polymermolekül bestrahlt.

Zusätzlich sei noch vermerkt, daß der Molekulargewichtsaufbau auch durch Bestrahlen mit Licht erfolgt, dessen Wellenlänge größer 400 nm ist, also im sichtbaren Bereich liegt, so daß die Polymeren auch unter den Bedingungen der natürlichen Bewitterung ständig ihr Molekulargewicht erhöhen, wobei auch die Anwesenheit von UV-Absorbern dies nicht unterbindet.

Gegenstand der vorliegenden Erfindung ist somit auch ein Verfahren zur Modifizierung der erfindungsgemäß erhältlichen Polycarbonate mit Endgruppen der Formel (Ic) oder (Id), die UV-Absorber enthalten können, gegebenenfalls im Gemisch mit bekannten aromatischen Polycarbonaten, das dadurch gekennzeichnet ist, daß man diese mit ultraviolettem und/oder mit sichtbarem Licht bestrahlt, beispielsweise der natürlichen Bewitterung durch Sonnenlicht aussetzt.

Den erfindungsgemäß erhältlichen Polycarbonaten mit Endgruppen der Formel (Ic) oder (Id) , gegebenenfalls im Gemisch mit bekannten aromatischen Polycarbonaten, können außer oder anstelle von UV-Stabilisatoren andere, in der Polycarbonatchemie übliche Additive wie Gleitmittel, Entformungsmittel, Stabilisatoren gegen Hitze oder Feuchtigkeit, Füllstoffe, Verstärkungsstoffe, Flammschutzmittel, Farbstoffe, Pigmente und/oder Schlagzähmodifikatoren vor oder während der Formgebung der Polycarbonate in den üblichen Mengen zugesetzt werden. Hierbei können an Füllstoffen oder Verstärkungsstoffen beispielsweise 10 bis 50 Gew.-%, bezogen auf das Gesamtgewicht der Formmassen, an Glasfasern, Glaskugeln, Glimmer, Silikaten, Feldspat, Quarz, $TiO_2$ oder/und Wollastonit eingesetzt werden.

Durch das Bestrahlen von Lösungen der erfindungsgemäß erhältlichen Polycarbonate mit Endgruppen der Formel (Ic) oder (Id) können Lösungen von sehr hochmolekularen Polycarbonaten hergestellt werden, aus denen Gießfolien für Elektroisolierzwecke erhalten werden können. Somit kann man von Polycarbonaten ausgehen, die in üblichen Anlagen für das Phasengrenzflächenverfahren erhalten werden, und deren Lösungen nach der üblichen Isolierung und Reinigung dann durch Bestrahlen gemäß vorliegender Erfindung in einfacher Weise weiterbehandelt werden können.

Die erfindungsgemäß erhältlichen Polycarbonate bzw. Polycarbonatgemische können vor oder nach Bestrahlung als Formkörper, insbesondere als Folien oder Oberflächenbeschichtungen, überall dort eingesetzt werden, wo sie Bestrahlung ausgesetzt sind wie dem Sonnenlicht bei der Bewitterung. Hierbei fungieren die erfindungsgemäßen Endgruppen der Formel (Ic) oder (Id) auch als UV-Absorter und können somit den Schutz gegen UV-Strahlen, gegebenenfalls mit den zugesetzten UV-Absorbern, bewirken.

Dies ist beispielsweise der Fall bei außenliegenden Teilen im Bau- und Automobilsektor, insbesondere bei glasklaren Formkörpern und Folien.

Beispielhaft sei die Verscheibung mit Hohlkammerplatten ( Stegdoppelplatten) bei Gewächshäusern genannt. Die erfindungsgemäß erhältlichen Polycarbonate bzw. Polycarbonatgemische können insbesondere dort eingesetzt werden, wo die Verarbeitung niedrigviskoser Typen zweckmäßig ist, also beispielsweise zur Herstellung dünnwandiger Formteile, sofern diese nachfolgend entweder thermisch oder photochemisch nachbehandelt werden können.

**Beispiel 1**

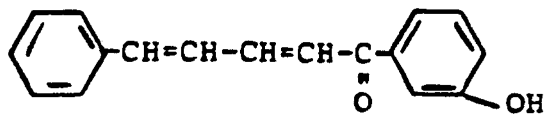

In eine Lösung von 40 g (1 Mol) NaOH in einer Mischung aus 300 ml Wasser und 150 ml Ethanol werden 66,1 g (0,5 Mol) Zimtaldehyd und 68,1 g (0,5 Mol) 3-Hydroxy-acetophenon bei 25°C eingetragen. Unter Rühren wird 2 h bei 50°C umgesetzt, sodann über Nacht bei Raumtemperatur stehengelassen. Nach Zugabe des gleichen Volumens Wasser und Ansäuern mit konzentrierter Salzsäure wird das Produkt mit Methylenchlorid extrahiert, die organische Phase mit Wasser neutral gewaschen und am Rotationsverdampfer eingeengt. Der orange, kristalline Rückstand wurde aus Toluol umkristallisiert und ergab 44 g (35 % d. Th.) gelb orange Kristalle vom Schmelzpunkt 130 bis 132°C. berechnet: C 81,6 %, H 5,60 % gefunden: C 80,8 %, H 5,67 %.

**Beispiel 2**

Herstellung und Prüfung eines aromatischen Polycarbonats. In einer Lösung aus 45,7 g (0,2 Mol) Bisphenol A, 16 g (0,4 Mol) NaOH und 535 ml Wasser wird 2,0 g (0,008 Mol) des Cinnamyliden-3-hydroxy-acetophenons aus Beispiel 1 gelöst. Danach werden bei 20 bis 25°C unter Rühren 32 g (0,32 Mol) Phosgen eingeleitet.

Der pH-Wert wird durch die Zugabe von 25 ml 45 %-iger wäßriger Natronlauge auf pH 12 gehalten. Nach beendeter Phosgenierung wird der pH-Wert durch Zugabe von weiteren 20 ml der 45 %-igen Natronlauge auf pH 13-14 angehoben, 4 ml wäßrige, 4 %-ige Triethylaminlösung zugefügt und weitere 45 Minuten gerührt. Die untere, organische Phase wird abgetrennt, angesäuert, elektrolytfrei gewaschen und eingeengt. Nach 15 h Trocknung bei 80°C im Vakuumtrockenschrank wurde ein gelbes Produkt mit der relativen Lösungsviskosität η rel 1,308 (an einer 0,5 %-igen Lösung in Methylenchlorid gemessen) erhalten.

Eine 10 %-ige Lösung des Polycarbonats wurde in Methylenchlorid mit einer UV-Lampe (Typ HPK 125 W) bestrahlt und davon 8-stündlich eine Probe gezogen, deren rel. Lösungsviskosität (gemessen wie vorstehend) bestimmt wurde.

| Zeit (h) | η rel |
|---|---|
| 0 | 1,308 |
| 8 | 1,508 |
| 16 | 1,703 |
| 24 | 1,899 |
| 32 | 1,962 |
| 40 | 2,062 |
| 48 | 2,235 |
| 56 | 2,447 |
| 64 | 2,546 |
| 72 | 2,599 |

Das Produkt war nach 72 h aufgehellt und trotz des hohen Molekulargewichts noch vollständig und homogen gelöst, woraus man auf linearen Molekulargewichtsaufbau schließen kann.

Zur Prüfung des Aufbauverhaltens in der Festphase wird aus dem Polycarbonat ein ca. 0,1 mm dicker Film gegossen und hinter einer Doppelglasscheibe dem Tageslicht ausgesetzt.

| Tage | η rel |
|---|---|
| 0 | 1,311 |
| 8 | 1,290 |
| 15 | 1,296 |
| 22 | 1,313 |
| 30 | 1,341 |
| 38 | 1,365 |

Man erkennt, daß das Molekulargewicht nach einer Induktionsperiode sogar im Glas-gefilterten Sonnenlicht deutlich ansteigt.

Zur Prüfung auf das thermische Verhalten wurden jeweils 2 g des Polycarbonats in 20 ml Methylenchlorid gelöst, und das Lösungsmittel in 100 ml Rundkolben bei etwa 40°C im Wasserstrahlvakuum am Rotavapor abgezogen. Die Kolben mit dem so auf der Innenwand erzeugten Polycarbonatfilm wurden dann in Metallbäder getaucht.

Aus Tabelle 1 sieht man den bei unterschiedlichen Temperaturen mit der Zeit zunehmenden Molekulergewichtsaufbau des Polycarbonats.

**Tabelle 1** ($\eta_{rel}$ gemessen wie vorstehend)

| Temperatur | (z. Vgl.) | | Temperungszeit/min | | |
|---|---|---|---|---|---|
| °C | 0 | 5 | 15 | 30 | 60 |
| 280 | | | 1,330 | | |
| 290 | | | 1,316 | | |
| 300 | (1,308) | 1,316 | 1,327 | 1,334 | 1,448 |
| 310 | | | 1,357 | | |

**Patentansprüche**

1. Verfahren zur Herstellung von aromatischen Polycarbonaten mit konjugierte Doppelbindungen enthaltenden Endgruppen und mit mittleren Molekulargewichten $\tilde{M}w$ (Gewichtsmittel) zwischen 2 000 und 100 000, vorzugsweise zwischen 5000 und 80 000, besonders bevorzugt zwischen 10 000 und 50 000, gegebenenfalls im Gemisch mit bekannten aromatischen Polycarbonaten mit $\tilde{M}w$ (Gewichtsmittel) zwischen 2 000 und 100 000, vorzugsweise zwischen 5000 und 80 000, besonders bevorzugt zwischen 10 000 und 50 000 aus Diphenolen, 0,5 bis 40 Mol-%, vorzugsweise 1 bis 20 Mol-%, besonders bevorzugt 2 bis 10 Mol-%, bezogen auf Mole Diphenole, Kettenabbrechern und Phosgennach den bekannten Polycarbonatherstellungsmethoden des Phasengrenzflächenverfahrens oder des Verfahrens in homogener Lösung, dadurch gekennzeichnet, daß man Kettenabbrecher der Formel (Ia) oder (Ib)

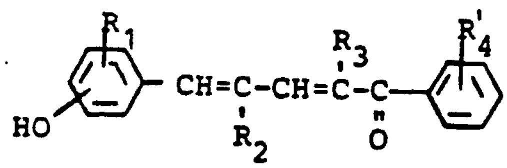

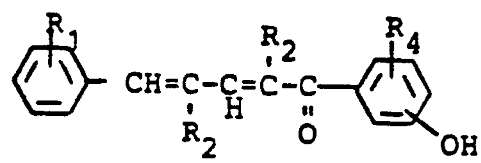

gegebenenfalls in Kombination mit maximal gleichen Molmengen an anderen, bekannten Kettenabbrechern verwendet, wobei

$R_1$ bis $R_4$ gleich oder verschieden sind und H, $CH_3$, $C_2H_5$, n-$C_3H_7$, iso-$C_3H_7$, n-$C_4H_9$, iso-$C_4H_9$, sec-$C_4H_9$, $C_6H_5$ oder Cl sein können.

2. Verfahren gemäß Anspruch 1, dadurch gekennzeichnet, daß man unter den Polycarbonatherstellungsbedingungen cokondensierbare bifunktionelle Oligomere oder Polymere mit Molekulargewichten $\bar{M}n$ (Zahlenmittel) zwischen 500 und 10 000 in Mengen von bis zu 450 Gew.-%, bezogen auf die Gewichtsmenge an eingesetzten monomeren Diphenolen, mitverwendet.

3. Aromatische Polycarbonate mit $\bar{M}w$ zwischen 2000 und 100 000 mit konjugierte Doppelbindungen enthaltenden Endgruppen der Formel (Ic) oder (Id)

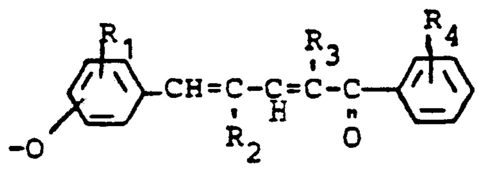

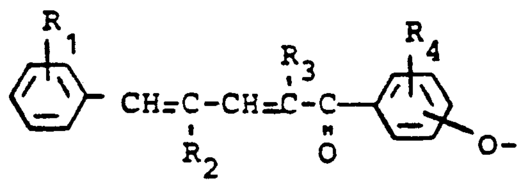

worin

$R_1$ bis $R_4$ die für Formel (Ia/Ib) genannte Bedeutung haben,

gegebenenfalls im Gemisch mit bekannten aromatischen Polycarbonaten mit $\bar{M}w$ zwischen 2000 und 100 000, die aus dem Kettenabbruch mit den anderen, bekannten Kettenabbrechern resultieren, erhältlich gemäß Verfahren der Ansprüche 1 und 2.

4. Polycarbonate mit mittleren Molekulargewichten $\bar{M}w$ (Gewichtsmittel ermittelt durch Lichtstreuung) zwischen 2000 und 100 000 der Formel (II)

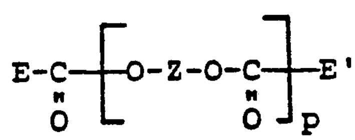

worin -O-Z-O ein Diphenolatrest ist,

E und E' gleich oder verschieden sind und wobei mindestens einer der Reste E oder E' einem Rest der Formel (Ic) oder (Id) entspricht, worin $R_1$ bis $R_4$ die für Formel (Ia/Ib) genannte Bedeutung haben, und wobei die übrigen Endgruppen E und E' aus der Reaktion mit den anderen, bekannten phenolischen Kettenabbrechern resultieren und wobei "p" der Polymerisationsgrad ist, der aus den Molekulargewichten $\bar{M}w$ von 2000 bis 100 000 resultiert.

5. Verfahren zur Modifizierung der Polycarbonate der Ansprüche 3 und 4, dadurch gekennzeichnet, daß man diese mit ultraviolettem und/oder sichtbarem Licht bestrahlt.

6. Verfahren zur Modifizierung der Polycarbonate gemäß Anspruch 5, dadurch gekennzeichnet, daß diese UV-Absorber enthalten.

7. Verfahren gemäß Ansprüche 5 und 6, dadurch gekennzeichnet, daß die Polycarbonate der natürlichen Bewitterung durch Sonnenlicht ausgesetzt werden.

8. Verbindungen der Formel (Ia/b)

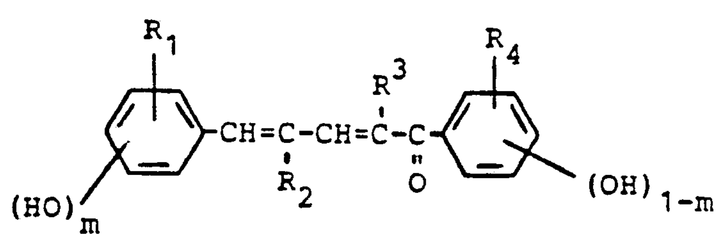

worin
$R_1$ bis $R_4$ gleich oder verschieden sein können und H, $CH_3$, $C_2H_5$, n-$C_3H_7$, iso-$C_3H_7$, n-$C_4H_9$, iso-$C_4H_9$, sec-$C_4H_9$, $C_6H_5$ oder Cl sein können und "m"0 oder 1 sein kann.

9. Verfahren zur Herstellung der Verbindungen der Formel I(a/b) des Anspruchs 8, dadurch gekennzeichnet, daß man Zimtaldehyde der Formal (Ie)

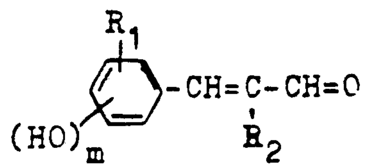

(Ie)

mit Acetophenonen der Formel (If)

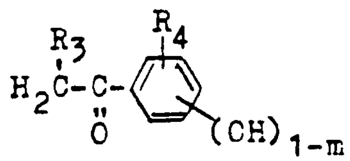

(If),

worin $R_1$ bis $R_4$ und "m" die in Anspruch 8 genannte Bedeutung haben, in Alkohol/Wasser - Gemischen bei Temperaturen von 20°C bis 100°C und Reaktionszeiten von etwa 30 Minuten bis etwa 3 Stunden umsetzt, danach das erhaltene Reaktionsgemisch ansäuert, mit einem organischen, nicht mit Wasser mischbaren Lösungsmittel extrahiert und den Extrakt in bekannter Weise aufarbeitet.

**Claims**

1. A process for the production of aromatic polycarbonates containing terminal groups with conjugated double bonds and having average molecular weights $\bar{M}w$ (weight average) of 2,000 to 100,000, preferably of 5,000 to 80,000 and more preferably of 10,000 to 50,000, optionally in admixture with known aromatic polycarbonates having molecular weights $\bar{M}w$ (weight average) of 2,000 to 100,000, preferably of 5,000 to 80,000 and more preferably of 10,000 to 50,000 of diphenols, 0.5 to 40 mole %, preferably 1 to 20 mole % and more preferably 2 to 10 mole %, based on moles didiphenols, of chain terminators and phosgene by the known interfacial method process for producing polycarbonates or by the method in homogeneous solution, characterized in that chain terminators corresponding to formulae (Ia) or (Ib)

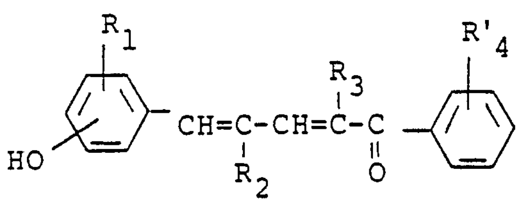

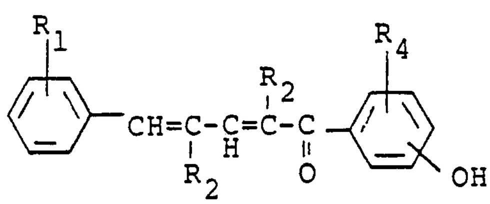

in which $R_1$ to $R_4$ may be the same or different and represent H, $CH_3$, $C_2H_5$, n-$C_3H_7$, iso-$C_3H_7$, n-$C_4H_9$, iso-$C_4H_9$, sec-$C_4H_9$, $C_6H_5$ or Cl, are used, optionally in combination with at most the same molar quantities of other known chain terminators.

2. A process as claimed in claim 1, characterized in that bifunctional oligomers or polymers co-condensable under the polycarbonate production conditions and having molecular weights M̄n (number average) of 500 to 10,000 are used in quantities of up to 450 % by weight, based on the quantity by weight of monomeric diphenols used.

3. Aromatic polycarbonates having molecular weights M̄w of 2,000 to 100,000 and containing terminal groups with conjugated double bonds corresponding to formulae (Ic) or (Id)

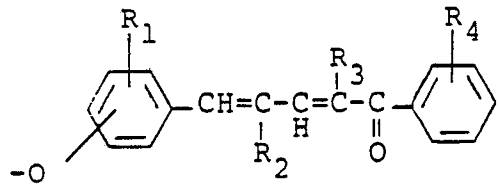

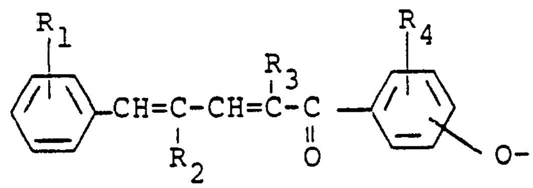

in which $R_1$ to $R_4$ have the meanings defined for formula (Ia/Ib), optionally in admixture with known aromatic polycarbonates having M̄w values of 2,000 to 100,000, which result from the chain termination with other known chain terminators, obtainable by the process claimed in claims 1 and 2.

4. Polycarbonates having average molecular weights M̄w (weight average, as determined by light scattering) of 2,000 to 100,000 corresponding to formula (II)

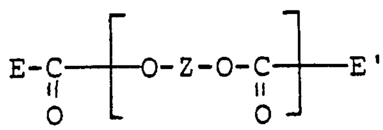

in which -O-Z-O is a diphenolate residue, E and E' may be the same or different and at least one of the groups E or E' corresponds to a group of formula (Ic) or (Id), in which $R_1$ to $R_4$ have the meanings defined for formula (Ia/Ib), the other terminal groups E and E' resulting from the reaction with the other known phenolic chain terminators, and "p" is the degree of polymerization resulting from the molecular weights M̄w of 2,000 to 100,000.

5. A process for modifying the polycarbonates claimed in claims 3 and 4, characterized in that they are exposed to ultraviolet and/or visible light.

6. A process for modifying the polycarbonates claimed in claim 5, characterized in that the polycarbonates contain UV absorbers.

7. A process as claimed in claims 5 and 6, characterized in that the polycarbonates are exposed to natural weathering by sunlight.

8. Compounds corresponding to the following formula

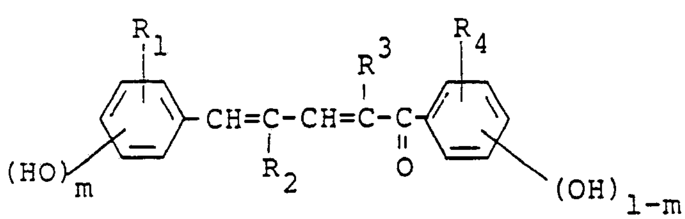

(Ia/b)

in which $R_1$ to $R_4$ may be the same or different and represent H, $CH_3$, $C_2H_5$, n-$C_3H_8$, iso-$C_3H_7$, n-$C_4H_9$, iso-$C_4H_9$, sec-$C_4H_9$, $C_6H_5$ or Cl and "m" is 0 or 1.

9. A process for the production of the compounds corresponding to formula (Ia/b) in claim 8, characterized in that cinnamic aldehydes corresponding to the following formula

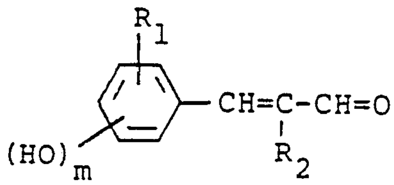
(Ie)

are reacted with acetophenones corresponding to the following formula

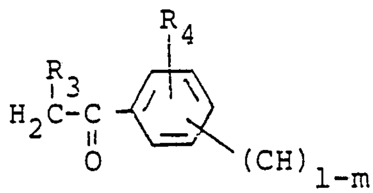
(If)

in which $R_1$ to $R_4$ and "m" have the meanings defined in claim 8 in mixtures of alcohol and water at temperatures of 20°C to I100°C and over reaction times of from about 30 minutes to about 3 hours, the reaction mixture obtained is subsequently acidified, extracted with an organic water-immiscible solvent and the extract worked up in known manner.

**Revendications**

1. Procédé de production de polycarbonates aromatiques porteurs de groupes terminaux contenant des doubles liaisons conjuguées et de poids moléculaires moyens M̄p (moyennes en poids) compris entre 2000 et "100 000, de préférence entre 5000 et 80 000, notamment entre 10 000 et 50 000, le cas échéant en mélange avec des polycarbonates aromatiques connus de poids moléculaire M̄p (moyennes en poids) compris entre 2000 et 100 000, de préférence entre 5000 et 80 000, notamment entre 10 000 et 50 000 à partir de diphénols, de 0 5 à 40 moles % , de préférence 1 à 20 moles %, notamment 2 à 10 moles %, par rapport aux moles de diphénols, d'agents de terminaison de chaîne et de phosgène selon les opérations connues de production de polycarbonates par le procédé à l'interface entre phases ou par le procédé en solution homogène, caractérisé en ce qu'on utilise des agents de terminaison de chaîne de formule (Ia) ou (Ib)

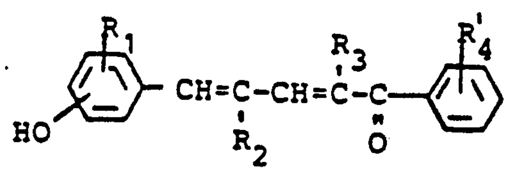

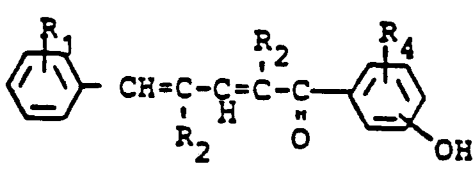

le cas échéant en association avec des quantités molaires au maximum égales d'autres agents connus de terminaison de chaîne, $R_1$ à $R_4$ etant identiques ou différents et pouvant représenter H, $CH_3$, $C_2H_5$, n-$C_3H_7$, iso-$C_3H_7$, n-$C_4H_9$, iso-$C_4H_9$, sec.-$C_4H_9$, $C_6H_5$ ou Cl.

2. Procédé suivant la revendication 1, caractérisé en ce qu'on utilise s imultanément des oligomères ou polymères bifonctionnels co-condensables dans les conditions de production de polycarbonates, de poids moléculaires M̄n (moyennes en nombre) compris entre 500 et 10 000, en quantités allant jusqu' à 450 % en poids par rapport à la quantité en poids de diphénols monomériques utilisés.

3. Polycarbonates aromatiques de Mp compris entre 2000 et 100 000, porteurs de groupes terminaux contenant des doubles liaisons conjuguées de formule (Ic) ou (Id)

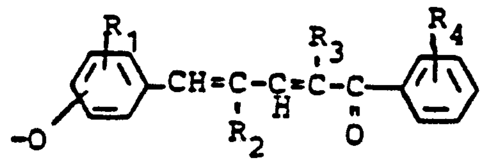

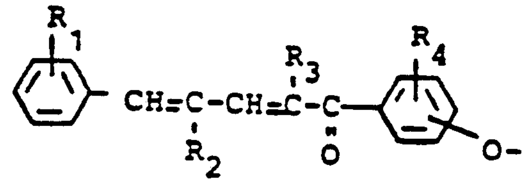

où $R_1$ à $R_4$ ont la définition mentionnée pour la formule (Ia/Ib), le cas échéant en mélange avec des polycarbonates aromatiques connus de M̄p compris entre 2000 et 100 000, qui résultent de la terminaison de chaîne avec les autres agents connus de terminaison de chaîne, pouvant être obtenus conformément au procédé suivant les revendications 1 et 2.

4. Polycarbonates de poids moléculaires moyens M̄p (moyennes en poids déterminées par diffusion de la lumière) entre 2000 et 100 000, de formule (II)

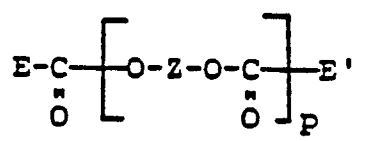

dans laquelle -O-Z-O est un reste diphénolate, E et E' sont identiques ou différents et l'un au moins des restes E et E' correspond à un reste de formule (Ic) ou (Id), où $R_1$ à $R_4$ ont la définition mentionnée pour la formule (Ia/Ib), et les autres groupes terminaux E et E' résultent de la réaction avec les autres agents phénoliques connus de terminaison de chaîne et "p" désigne le degré de polymérisation qui résulte des poids moléculaires M̄p de 2000 à 100 000.

5. Procédé de modification des polycarbonates des revendications 3 et 4, caractérisé en ce qu'on irradie ces polycarbonates avec de la lumière ultraviolette et/ou visible.

6. Procédé de modification des polycarbonates suivant la revendication 5, caractérisé en ce que ces polycarbonates contiennent des agents absorbant les rayons ultraviolets.

7. Procédé suivant les revendications 5 et 6, caractérisé en ce que les polycarbonates sont exposés au vieillissement naturel par la lumière solaire.

8. Composés de formule (Ia/Ib)

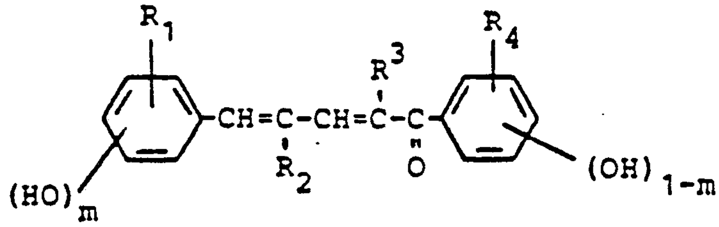

dans laquell $R_1$ à $R_4$ peuvent être identiques ou différents et peuvent représenter H, $CH_3$, $C_2H_5$, n-$C_3H_7$, iso-$C_3H_7$, n-$C_4H_9$, iso-$C_4H_9$, sec.-$C_4H_9$, $C_6H_5$ ou Cl et "m" peut avoir la valeur 0 ou 1.

9. Procédé de production' des composés de formule I(a/b) suivant la revendication 8, caractérisé en ce qu'on fait réagir des cinnamaldéhydes de formule (Ie)

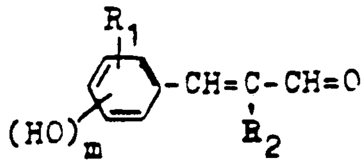

(Ie)

avec des acétophénones de formule (If)

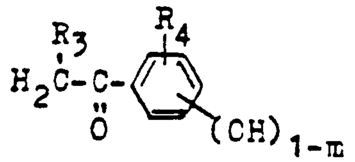

(If),

où $R_1$ à $R_4$ et "m" ont la définition mentionnée dans la revendication 8, dans des mélanges alcool/eau à des températures de 20 à 100°C et pendant des durées de réaction d'environ 30 minutes à environ 3 heures, puis on acidifie le mélange réactionnel obtenu, on l'extrait avec un solvant organique non miscible à l'eau et on traite l'extrait d'une manière connue.